# EUROPEAN PATENT APPLICATION

(11) **EP 3 141 189 A1**
(43) Date of publication of application: **15.03.2017**
(21) Application number: 15185058.3
(22) Date of filing: 14.09.2015
(51) Int. Cl.: A61B 6/04, A61B 6/08, A61B 6/00

(54) **A TECHNIQUE FOR ALIGNING FIELD OF VIEW OF A MEDICAL DEVICE WITH A DESIRED REGION ON A SUBJECT'S BODY**

(71) Applicant: Siemens Aktiengesellschaft, 80333 München (DE)
(72) Inventor: Groten, Raphaela, 80469 München (DE); Johnson, Rebecca, 81825 München (DE)

(57) **Abstract**

A system and method for aligning a field of view (FOV) of a movable part of a medical device with a desired region on a subject's body is presented. In the system, a position detecting unit detects an identifiable optical front projected on the subject's body and at least partially overlapping with the desired region and generates information corresponding to a spatial position of the identifiable optical front. From the information, a processing module determines the spatial position of the identifiable optical front with respect to a known position of the FOV and generates an instruction set that corresponds to one or more mechanical adjustments of the movable part to change a position of the FOV from the known position to the spatial position of the identifiable optical front. An executing module directs a moving mechanism of the movable part to carry out mechanical motions according to the instruction set.

## Description

The present invention relates to medical devices, and more particularly to a system and a method of aligning a field of view of a movable part of a medical device with a desired region on a body of a subject.

Medical technology in recent times has witnessed advent of numerous medical devices. A lot of these medical devices are with movable parts that are used by aligning with a desired region on a subject's body, for example imaging devices, medical radiation devices, etc. In imaging devices such a X-Ray devices the movable part is a source from where the X-Rays are projected on a specific part on the body of a subject in order to get an X-ray image of that part. Usually the subject is positioned on a table and the movable part is moved mechanically or by help of moving techniques operated by an operator and the movable part is aligned on top of or in front of the desired region to be imaged. Similarly, in radiation devices, alignment of a movable part which acts as a source of radiation is done before a desired part on the subject's body is irradiated.

More concrete examples of such medical devices which have a movable part are C-arm based imaging devices. In these devices the C-arm is moved along several axis of movement compared to a subject laying on a table for example if say an axis if formed in a direction from head to toe of a subject positioned in a lying posture on the table of the device, then as an example, to align the C-arm heads with respect to a desired part of the subject's body, the C-arm movements are translational motions in x, y and z axis directions mutually perpendicular to each other. More movement may be possible in form of rotation of the C-arm about the axis formed by the subject positioned on the patient's bed or patient's table. In present times the aforementioned movements are implemented by mechanically moving the C-arm or by manually operating motors that move the C-arm to a desired orientation in order to align the C-arm with the desired part of the subject's body i.e. to orient the C-arm such that a field of view of the C-arm from which radiations are directed towards the subject is brought in with a desired region on the body of the subject. Manually moving the C-arm or manually operating motors that move the C-arm requires expertise of the operator. Moreover the alignment may not be accurate.

Thus the object of the present technique is to provide a technique, a system and a method, for aligning a field of view of a movable part of a medical device with a desired region on a body of a subject, and which at least partially obviates human intervention and thus possibilities of misalignment.

The above objects are achieved by a system for aligning a field of view of a movable part of a medical device with a desired region on a body of a subject according to claim 1 and a method for aligning a field of view of a movable part of a medical device with a desired region on a body of a subject according to claim 13 of the present technique. Advantageous embodiments of the present technique are provided in dependent claims. Features of claim 1 may be combined with features of dependent claims dependant on claim 1, and features of dependent claims can be combined together. Similarly, features of claim 13 may be combined with features of dependent claims dependant on claim 1, and features of dependent claims can be combined together.

According to a first aspect of the present technique, a system for aligning a field of view with a desired region is presented. The field of view is of a movable part of a medical device. The desired region is on a body of a subject. The alignment by the system is performed from an identifiable optical front projected on the body of the subject such that the identifiable optical front at least partially overlaps with the desired region on the body of the subject. The system includes a position detecting unit, a processing module and an executing module.

The position detecting unit detects the identifiable optical front while the identifiable optical front at least partially overlaps with the desired region on the body of the subject. The position detecting unit generates information corresponding to a spatial position of the identifiable optical front.

The processing module receives the information from the position detecting unit and determines the spatial position of the identifiable optical front with respect to a known position of the field of view of the movable part of the medical device. The processing module then generates an instruction set. The instruction set corresponds to one or more mechanical adjustments of the movable part to change a position of the field of view of the movable part from the known position to the spatial position of the identifiable optical front.

The executing module receives the instruction set and directs a moving mechanism of the movable part to carry out mechanical motions according to the instruction set.

Thus, with the system of the present technique the alignment of the moveable part is achieved without manual intervention.

In an embodiment of the system, the system includes a pointer to project the identifiable optical front on the body of the subject. The pointer may be adapted to project a predefined identifiable optical front on the subject's body and the position detecting unit may be adapted to detect only the predefined identifiable optical front projected by the pointer, thus making the system more specific and secure.

In another embodiment of the system, the pointer includes a lock module. The lock module is changeable between a first state and a second state. The pointer communicates to the position detecting unit a state indication. The position detecting unit detects the identifiable optical front when the state indication from the pointer communicates that the lock module is in the first state. Thus, the position detecting unit detects the identifiable optical front only when an operator sets the lock module in the first state. Thereby the operator has a control on the initiation of the system.

In another embodiment of the system, the pointer is a spatially coherent light source. Thus specifically defined identifiable optical front is projected on the body of the subject.

In another embodiment of the system, the identifiable optical front is a point. Thus the FOV of the movable part of the medical device may be aligned in such a way that it is focused around a specifically pinpointed location on the subject's body.

In another embodiment of the system, the identifiable optical front is an extended area having a predefined shape. Thus the FOV of the movable part of the medical device may be aligned with an extended part as the desired region on the subject's body.

In another embodiment of the system, the position detecting unit detects a distortion of the predefined shape of the identifiable optical front while the identifiable optical front at least partially overlaps with the desired region on the body of the subject. The position detecting unit further generates data corresponding to the distortion of the predefined shape of the identifiable optical front. The processing module receives the data from the position detecting unit and determines an angle of illumination of the identifiable optical front. The angle of illumination corresponds to an angle, with respect to the desired region, from which the identifiable optical front is projected on the desired region. The processing module further generates a second instruction set. The second instruction set corresponds to one or more further mechanical adjustments of the movable part in order to attain an aligned position of the movable part. The aligned position of the movable part is at an aligned angle with respect to the desired region. The aligned angle is same as the angle of illumination. In this embodiment of the system, the executing module receives the second instruction set and directs the moving mechanism of the movable part to carry out mechanical motions according to the second instruction set. Thus the movable part is aligned in such a way that any radiation from the movable part is directed in a particular angle with respect to the desired region i.e. exposure of the desired region is obtained from a particular spatial angle with respect to the desired region.

In another embodiment of the system, the position detecting unit, the processing module, and the executing module are in wireless communication with each other. Thus the parts of the system may be set up remote from each other and without wired connections - giving more flexibility to implementation of the system.

In another embodiment of the system, the position detecting unit acquires an image of the body with respect to a patient's table of the medical device. The processing module determines from the image an orientation of the body with respect to the patient's table. The executing module directs the moving mechanism of the movable part to carry out mechanical motions according to the instruction set when the orientation of the body is a desired orientation. Thus chance of collision between any part of the body of the subject with a part of the movable part of the medical device is reduced.

In another embodiment of the system, the system further includes a mechanical control module. The mechanical control module effects minor adjustments in mechanical motions carried out by the movable part. The mechanical control module is mechanically operable by an operator. Thus minor adjustments may be done allowing finer alignment desired by the operator.

In another embodiment of the system, the processing module generates a table-movement instruction. The table-movement instruction is for moving the patient's table. The executing module receives the table-movement instruction and directs a moving mechanism of the patient's table to carry out mechanical motions according to the table-movement instruction. Thus the patient's table is moved to help alignment of the movable part's FOV with the desired region on the body of the subject, giving a greater range of movements which finally result in better alignment.

In another embodiment of the system, the executing module is integrated within the medical device. Thus the system may be implemented by using a processor of the medical device to perform as the executing module.

According to another aspect of the present technique, a method for aligning a field of view a field of view with a desired region is presented. The field of view is of a movable part of a medical device. The desired region is on a body of a subject. In the method, an identifiable optical front is projected on the body of the subject such that the identifiable optical front at least partially overlaps with the desired region on the body of the subject. Subsequently, the identifiable optical front is detected while the identifiable optical front at least partially overlaps with the desired region on the body of the subject. Then, information corresponding to a spatial position of the identifiable optical front is generated. Thereafter, the spatial position of the identifiable optical front with respect to a known position of the field of view of the movable part is determined. Next in the method, an instruction set is generated. The instruction set corresponds to one or more mechanical adjustments of the movable part to change a position of the field of view of the movable part from the known position to the spatial position of the identifiable optical front. Finally, a moving mechanism of the movable part is directed to carry out mechanical motions according to the instruction set.

Thus, with the method of the present technique the alignment of the moveable part is achieved without manual intervention.

In an embodiment of the method, the identifiable optical front is an extended area having a predefined shape. Thus the FOV of the movable part of the medical device may be aligned with an extended part as the desired region on the subject's body.

In another embodiment of the method, the method further includes detecting a distortion in the predefined shape of the identifiable optical front while the identifiable optical front at least partially overlaps with the desired region on the body of the subject. Subsequently, data corresponding to the distortion in the predefined shape of the identifiable optical front is generated. Next, an angle of illumination of the identifiable optical front is determined. The angle of illumination corresponds to an angle, with respect to the desired region, from which the identifiable optical front is projected on the desired region. Thereafter, a second instruction set is generated. The second instruction set corresponds to one or more further mechanical adjustments of the movable part to attain an aligned position of the movable part. At the aligned position the movable part is at an aligned angle with respect to the desired region. The aligned angle is same as the angle of illumination. Succeeding the previous step, the moving mechanism of the movable part is directed to carry out mechanical motions according to the second instruction set. Thus the movable part is aligned in such a way that any radiation from the movable part is directed in a particular angle with respect to the desired region i.e. exposure of the desired region is obtained from a particular spatial angle with respect to the desired region. In another embodiment of the method, in the method an image of the body with respect to a patient's table of the medical device is acquired. From the image so acquired, an orientation of the body with respect to the patient's table is determined. Thereafter, the moving mechanism of the movable part is directed to carry out mechanical motions according to the instruction set when the orientation of the body is a desired orientation. Thus chance of collision between any part of the body of the subject with a part of the movable part of the medical device is reduced.

In another embodiment of the method, the method further includes generating a table-movement instruction to move the patient's table. Subsequently, a moving mechanism of the patient's table is directed to carry out mechanical motions according to the table-movement instruction. Thus the patient's table is moved to help alignment of the movable part's FOV with the desired region on the body of the subject, giving a greater range of movements which finally result in better alignment.

The present technique is further described hereinafter with reference to illustrated embodiments shown in the accompanying drawing, in which:
- FIG 1: schematically illustrates an exemplary embodiment of a system for aligning a field of view (FOV) of a movable part of a medical device with a desired region on a body of a subject;
- FIG 2: schematically illustrates another exemplary embodiment of the system of FIG 1;
- FIG 3: schematically illustrates the FOV of the movable part of the medical device and the desired region on the body of the subject;
- FIG 4: schematically illustrates an identifiable optical front (IOF);
- FIG 5: schematically illustrates the IOF of FIG 4 projected on the body of the subject and overlapping with the desired region on the body of the subject;
- FIG 6: schematically illustrates another exemplary embodiment of the system;
- FIG 7: schematically illustrates an exemplary embodiment a pointer depicting a lock module in a first state;
- FIG 8: schematically illustrates the pointer of FIG 7 depicting the lock module in a second state;
- FIG 9: schematically represents an exemplary embodiment of the IOF in a point form;
- FIG 10: schematically represents an exemplary embodiment of the movable part depicting the FOV of the movable part aligned with the desired region on the body of the subject;
- FIG 11: schematically represents an exemplary embodiment of the IOF showing a distortion in the IOF;
- FIG 12: schematically represents another exemplary embodiment of the movable part depicting the FOV of the movable part aligned with the desired region on the body of the subject;
- FIG 13: schematically represents an exemplary embodiment of an undesirable orientation of the body of the subject with respect to the patient's table;
- FIG 14: schematically represents an exemplary embodiment of a desirable orientation of the body of the subject with respect to the patient's table; and
- FIG 15: depicts a flow chart illustrating an exemplary embodiment of a method for aligning a FOV of a movable part of a medical device with a desired region on a body of a subject; in accordance with aspects of the present technique.

Hereinafter, above-mentioned and other features of the present technique are described in details. Various embodiments are described with reference to the drawing, wherein like reference numerals are used to refer to like elements throughout. In the following description, for purpose of explanation, numerous specific details are set forth in order to provide a thorough understanding of one or more embodiments. It may be noted that the illustrated embodiments are intended to explain, and not to limit the invention. It may be evident that such embodiments may be practiced without these specific details.

It may be noted that in the present disclosure, the terms "first", "second", "third", etc. are used herein only to facilitate discussion, and carry no particular temporal or chronological significance unless otherwise indicated.

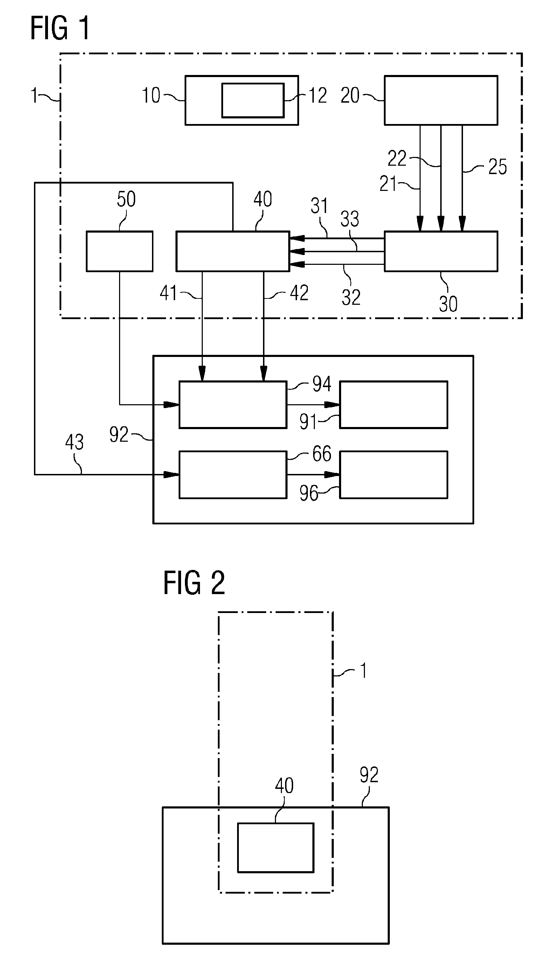

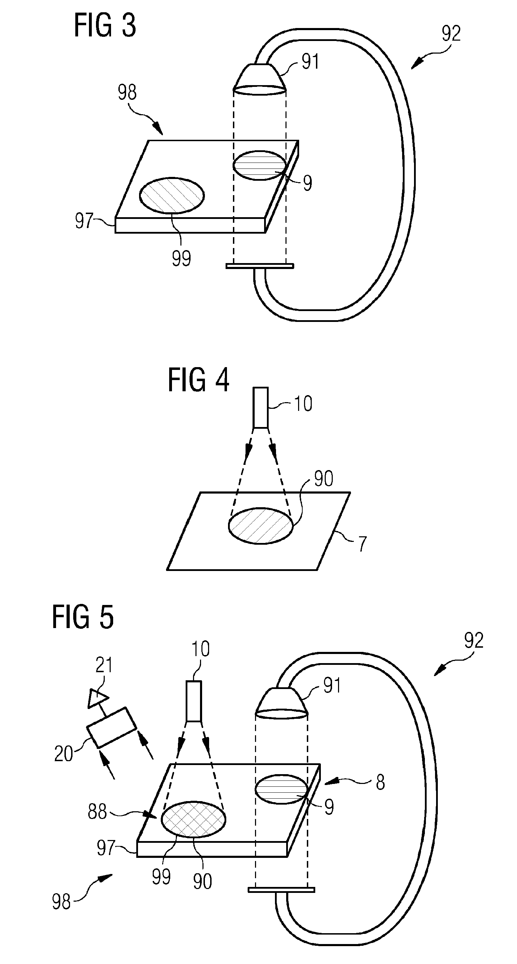

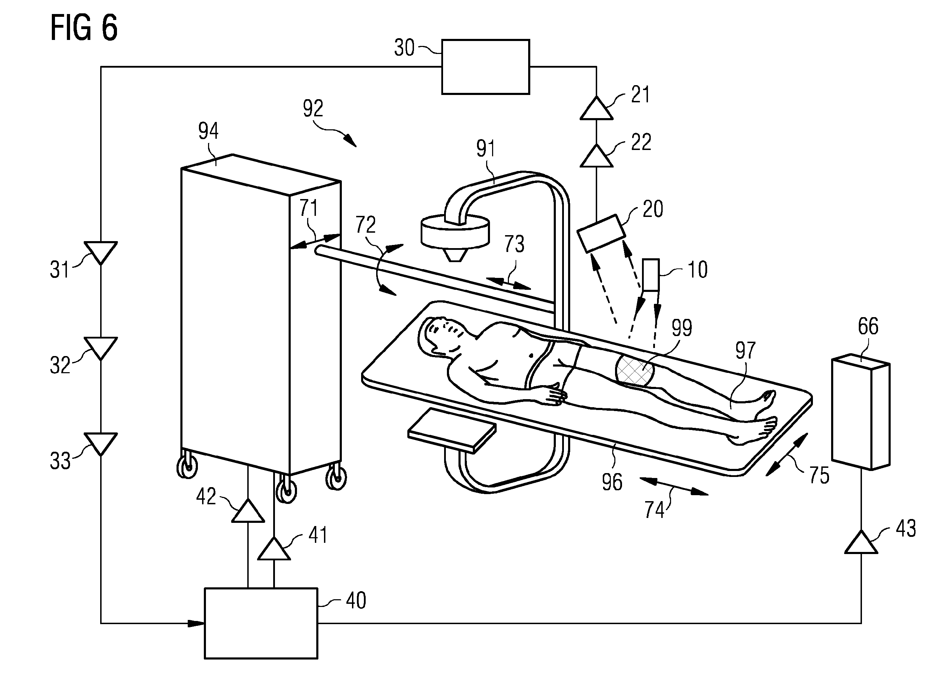

Referring to FIG 1, a system 1 is presented. FIG 1 has been explained hereinafter in combination with FIG 2-6. As seen schematically in FIGs 3 and 6, the system 1 is for aligning a field of view 9 (hereinafter the FOV 9) of a movable part 91 a medical device 92 with a desired region 99 on a body 97 of a subject 98. To understand more clearly, FIG 3 schematically shows the subject 98 with the body 97 also schematically represented. The function of the system 1 is to align the movable part 91 of the medical device 92 with the desired region 99 on the body 97 of the subject 98. To explain further, FIG 6 shows the subject 98 as a human being with the body 97 and the desired region 99 i.e. the part of the body 98 of the subject 98 with which the movable part 97 of the medical device 98 is to be aligned. The term 'align', and related terms, as used herein includes positioning the movable part 91 in relative orientation with the desired region 99 in such a way that the FOV 9 of the movable part 91 at least partially overlaps with the desired region 99. As seen in example of FIG 6, the movable part 91 is hovering over a chest region of the body 97 of the subject 98 such that the FOV 9 would probably be positioned at the chest of the subject 98, whereas the desired region 9 is at a knee of the subject 98. Thus the system 1 functions to perform movement of the movable part 91 such that the FOV 9 of the movable part 91 shifts to the desired region 9 by movement of the movable part 91 from over the chest of the subject 98 to the knee of the subject 98.

The system 1 functions by using an identifiable optical front 90, as depicted in FIGs 4 and 5. FIG 4 shows the identifiable optical front 90, hereinafter IOF 90, as projected on a plane 7. The IOF 90 may be understood as a region lit up by projections from a pointer 10. The pointer 10 may be a light source such as a spatially coherent source like laser source, a point source, or simply a flash light that projects lights up a defined area on the plane 7.

In the present technique, an operator (not shown), such as doctor or a X-ray operator, projects the IOF 90 on the body 97 of the subject 98 such that the IOF 90 at least partially overlaps with the desired region 99 on the body 97 of the subject 98, as depicted in FIGs 5 and 6 which depict the IOF 90 and the desired region 9 completely overlapping.

As shown in FIG 1, the system 1 includes the pointer 10, a position detecting unit 20, a processing module 30 and a executing module 40.

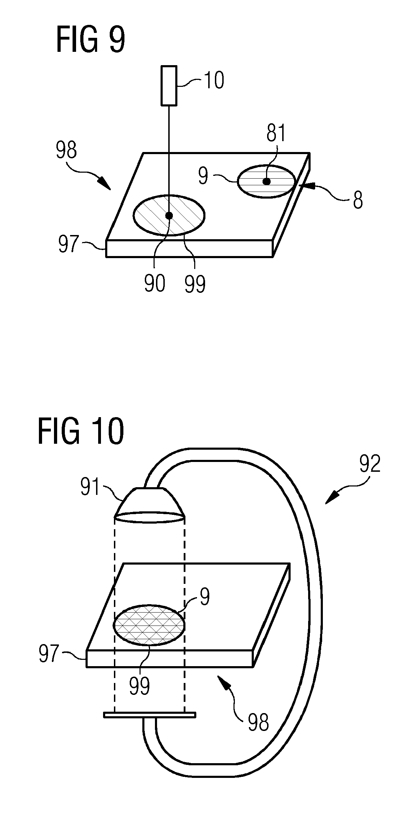

As shown in FIG 5 and 6, the pointer 10 projects the IOF 90 on the body 97 of the subject 98. The operator has to project the IOF 90 in such a way that the IOF 90 at least partially overlaps or is at least partially lights up the desired region 99 on the body 97 of the subject 98. The pointer 10 may include a spatially coherent light source 17. Furthermore the IOF 90 may in form of a point, as shown in FIG 9, or may be an extended area as shown in FIGs 4, 5 and 6. The extended area of the IOF 90 may have a predefined shape, for example a circle as shown in FIGs 4 and 5.

The position detecting unit 20, for example a camera, detects the IOF 90 while the IOF 90 at least partially overlaps with the desired region 99 on the body 97 of the subject 98. The position detecting unit 20, hereinafter PDU 20, may determine that the IOF 90 is at a partially overlapping position with the desired region 99 by either continuously monitoring the movements of the IOF 90 on the body 97 of the subject 98 and subsequently detecting the IOF 90 after the IOF 90 attains and holds a static position i.e. showing no movement over the body 97, for a predefined period of time. In this case the operator has to ensure that the IOF 90 comes to a static position for the predefined period of time when the IOF 90 is at least partially overlapping with the desired region 99 on the body 97 of the subject 98. In another embodiment of the system 1, as depicted in FIGs 1, 7 and 8 the pointer 10 may include a lock module 12.

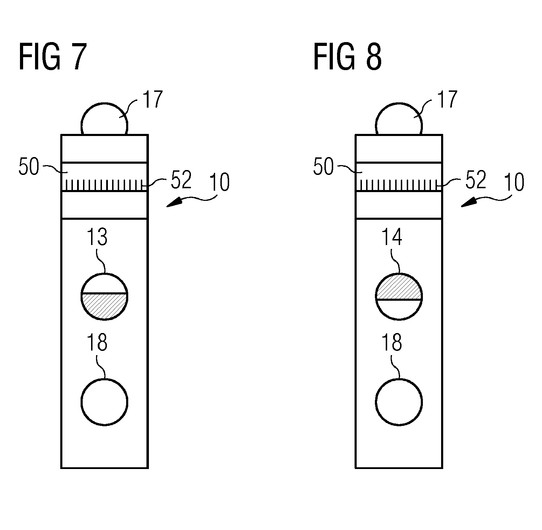

The lock module 12 is changeable between a first state 13 as depicted in FIG 7, and a second state 14 as depicted in FIG 8. The lock module 12 may be designed as integrated as a switch in the pointer 10 and may be alternated between the first state 13 and the second state 14 of the lock module 12 similar to 'ON' and 'OFF' states as standard functionality of a switch, wherein 'ON' state or mode may be understood as the first state 13 and the 'OFF' state may be understood as the second state 14 of the lock module 12. The switching of the lock module 12 between the first state 13 and the second state 14 is performed by the operator. The operator switches the lock module 12 from the second state 14 to the first state 13 when the IOF 90 is at least partially overlapping with the desired region 99 on the body 97 of the subject 98.

The pointer 10 is adapted to communicate to the PDU 20 when the lock module 12 is in the first state 13. The communication from the pointer 10 to the PDU 20 that the lock module 12 is in the first state 13 activates the PDU 20 to detect the IOF 90. The detection of the IOF 90 by the PDU 20 may be in form of acquiring image data of the IOF 90 with respect to the body 97 of the subject 98. Thus PDU 20 detects the IOF 90 when the state indication from the pointer 10 communicates that the lock module 12 is in the first state 13, which is an affirmation from the operator that the IOF 90 is at least partially overlapping with the desired region 99.

After detecting the IOF 90 while the IOF 90 is at least partially overlapping with the desired region 99, the PDU 20 generates information 21, as shown in FIGs 1, 5 and 6. The information 21 corresponds to a spatial position 88 of the IOF 90 as depicted particularly in FIG 5. The spatial position 88 is indicative of a position of the IOF 90 with respect to the body 97 of the subject 98. As shown in FIG 9, the IOF 90 may be in form of a point projected in the desired region 99 or as depicted in FIG 5 and 6, the IOF 90 may be an extended area overlapping with the desired region 99.

In system 1 as depicted in FIGs 1 and 6, the processing module 30 receives the information 21 from the PDU 20. With the information 21, the processing module 30, hereinafter referred to as the processor 30, extracts the spatial position 88 of the IOF 90 with respect to a known position 8 of the FOV 9 of the movable part 91 of the medical device 92. Thus the processor 30 knows the position 8 of the FOV 9 from the position of the movable part 91, for example as shown in FIG 6, the movable part 91 is at a position defined by the moving mechanism 94 i.e. how much different components (now shown) of moving mechanism 94 have moved from their default positions along the axes in X, Y, and Z direction, for example. The axes are represented, for example, by a first axis 71, a second axis 72 and a third axis 73. Thus by sensing the movements executed by components of the moving mechanism 94 from their default positions or orientations, the processor 30 is aware of the location or position of the movable part 91 and thus the processor 30 determines or knows the position 8 of the FOV 9 of the movable part 91 of the medical device 92. Thus, the processor 30 is possesses information regarding the position 8 of the FOV 9 and extracts the spatial position 88 of the IOF 90. The known position 8 and/or the spatial position 88 may be calculated or determined or known by the processor 30 by any known position identifying technique such as a co-ordinate system for example as co-ordinates in a three dimensional Cartesian co-ordinate system.

From the known position 8 of the FOV 9 and the spatial position 88 of the IOF 90, the processor 30 generates an instruction set 31. The instruction set 31 includes commands or directions to perform one or more mechanical adjustments of the movable part 91 to change a position of the FOV 9 from the known position 8 to the spatial position 88, for example, instruction set 31 includes commands or directions for the movement mechanism 94 to move the movable part 91 in such a way that the co-ordinates of the FOV 9 change from the known position 8 to the co-ordinates of the spatial position 88.

As shown in FIG 1 and 6, the system 1 includes the executing module 40. The executing module 40 may be present as part of the processor 30 or may be implemented as an additional function of the processor 30. The executing module 40 may, alternatively, be present as a separate unit that the processor 30 within the system 1. In an exemplary embodiment of the system 1, as depicted in FIG 2, the executing module 40 is integrated within the medical device 92. In one embodiment of the system 1, one or more of the PDU 20, the processor 30, and the executing module 40 are in wireless communication with each other and any exchange of communication or data or information or commands between them, for example the information 21 from the PDU 20 to the processor 30, is done wirelessly, for example by blue tooth or WiFi.

The executing module 40 receives the instruction set 31 and in turn directs, say as first directions 41, to the moving mechanism 94 of the movable part 91 to carry out mechanical motions according to the instruction set 31. As can be understood from a comparison of FIG 5 and FIG 10, as a result of the mechanical motions carried out by the moving mechanism 94 of the medical device 92 the movable part 91 changes position and the FOV 9 from the known position 8, as shown in FIG 5, is changed to a new position which is same as the spatial position 88 of the IOF 90, as depicted in FIG 10. The moving mechanism 94 may be a set of computer controller or electronically controlled and operated motors that carry out mechanical motions of the moving part 91 in different directions for example, as shown in FIG 6, along the axes 71, 72 and 73.

As aforementioned and depicted in FIG 9, in an exemplary embodiment of the system 1, the IOF 90 may be the point 90. In this embodiment, the processor 30 considers the known position 8 of the FOV 9 as position of a central point 81 present in the FOV 9. The processor 30 then in the instruction set 31 includes commands or directions to perform one or more mechanical adjustments of the movable part 91 to change the central point 81 of the FOV 9 from the known position 8 to the spatial position 88 of the point 90.

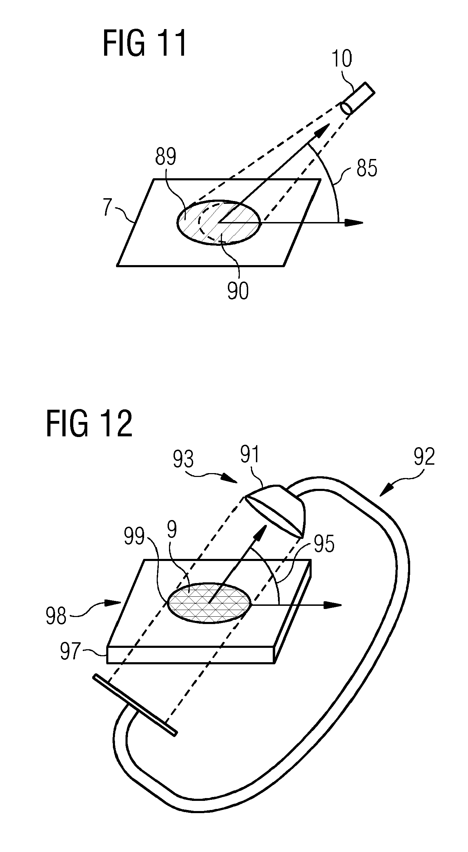

Now referring to FIG 11 and 12 in combination with FIG 1 to 10, another exemplary embodiment of the system 1 has been explained hereinafter. As aforementioned and depicted in FIG 4, the IOF 90 may be an extended area having a predefined shape. Now as depicted in FIG 11, if the operator shines or projects the IOF 90, at least partially overlapping with the desired region 99, from an angle with respect to the desired region 99, then owing to the angle and/or contour of the desired region 99, a distortion 89 as compared to the predefined shape of the IOF 90 will be developed in the IOF 90. The distortion 89 may be understood as a deviation from the predefined shape of the IOF 90 when projected normally on a flat surface such as the plane 7 of FIG 4.

The PDU 20 detects the distortion 89 of the predefined shape of the IOF 90 while the IOF 90 at least partially overlaps with the desired region 99 on the body 97 of the subject 98. The PDU 20 for example may detect the distortion 89 as image data. The PDU 20 subsequently generates data 22, as shown in FIGs 1 and 6, corresponding to the distortion 89 of the predefined shape of the IOF 90. The processor 30 receives the data 22 from the PDU 20. The processor 30 subsequently determines an angle of illumination 85 of the IOF 90 by comparing the predefined shape when without the distortion 88 and when with the distortion 88. The information of the predefined shape without distortion 88 may be provided to the processor 30 in advance or may be input into the processor 30 during the operation of the system 1. AS shown in FIG 11, the angle of illumination 85 corresponds to an angle, with respect to the desired region 99, from which the IOF 90 is projected on the desired region 99 by the operator. The operator may choose the angle from which the operator projects the IOF 90 upon the desired region 99 based on an angle at which the operator desires the FOV 9 to align with the desired region 99 or simply put if the movable part 91 is a X-ray source then depending on the angle at which the operator desired to acquire the X-ray image of the desired region 99.

As shown in FIG 1 and 6 in combination with FIGs 11 and 12, the processing module 30 after determining the angle of illumination 85 generates a second instruction set 32. The second instruction set 32 corresponds to one or more further mechanical adjustments of the movable part 91 to attain an aligned position 93 of the movable part 91, as shown in FIG 12. At the aligned position 93, the movable part 91 is at an aligned angle 95 with respect to the desired region 99 and the aligned angle 95 is same as the angle of illumination 85, which means that the movable part 91 is positioned at an angle with respect to the desired region 99 same as the angle from which the IOF 90 is projected by the pointer 10 on the desired region 99.

The executing module 40 receives the second instruction set 32 from the processor 30 and directs, for example as second directions 42 as shown in FIG 1 and 6, the moving mechanism 94 of the movable part 91 to carry out mechanical motions according to the second instruction set 32. Thus, if the medical device 92 is an X-ray imaging device and the movable part 91 is a C-arm of the X-ray imaging device, then the C-arm orients the X-ray source side of the C-arm at the same angle as the pointer 10 has projected the IOF 90 on the desired region 99.

Furthermore, in an exemplary embodiment of the system 1, as depicted in FIGs 1 and 6, based on the information 21 and/or the data 22, the processing module 30 generates a table-movement instruction 33 to move the patient's table 96. The generation of the table-movement instruction 33 may be understood to be similar to the generation of the instruction 31 as described aforementioned. The executing module 40 receives the table-movement instruction 33 and directs, in form of table-movement directions 43, a moving mechanism 66 of the patient's table 96 to carry out mechanical motions according to the patient's table instruction 33.

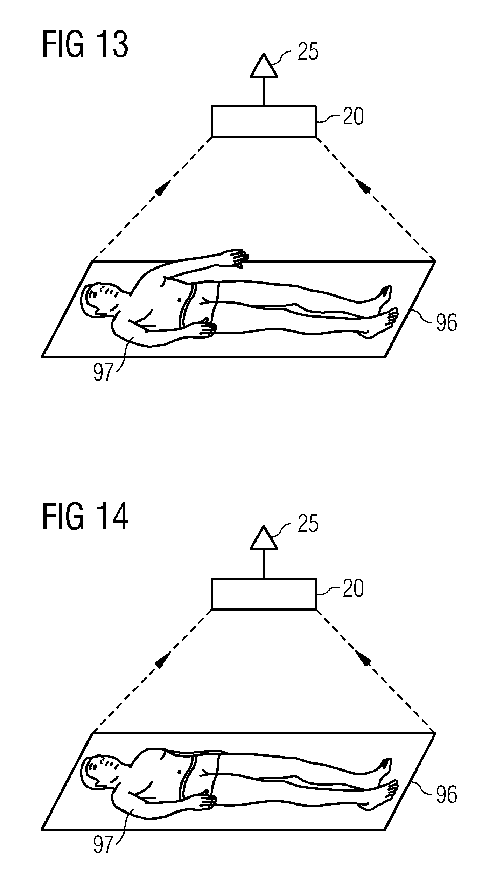

Referring now to FIGs 13 and 14 in combination with FIGs 1 and 6, another exemplary embodiment of the system 1 has been explained hereinafter. In this embodiment of the system 1, the PDU 20 acquires an image 25 of the body 97 with respect to the patient's table 96 of the medical device 92. The patient's table 96 may be understood as a surface on which the subject is laying down, seated or otherwise stationed during a medical procedure for which the medical device 92 is used. The processor 30 determines from the image 25 an orientation of the body 97 with respect to the patient's table 96. For example two orientations of the body 97 of the subject 98 are depicted schematically in FIGs 13 and 14. As shown in FIG 13 a part (not shown) of the body 97 is oriented or placed outside the patient's table 96, whereas as shown in FIG 14, all parts (not shown) of the body 97 are oriented within the patient's table 96. In this exemplary embodiment of the system 1, the executing module 40 directs the moving mechanism 94 of the movable part 91 to carry out mechanical motions according to the instruction set 31 and/or the second instruction set 32 when the orientation of the body 97 is a desired orientation which among FIGs 13 and 14 has been depicted in FIG 14.

Furthermore, as depicted in FIG 1, the system 1 includes a mechanical control module 50. The mechanical control module 50 effects minor adjustments in mechanical motions carried out by the movable part 91. Thus any finer adjustments to the alignment may be achieved after the moving mechanism 94 of the medical device 92 has aligned the movable part 91 according to the directions 41 from the executing module 40. The mechanical control module 50 is configured to be operated by an operator. As shown in FIGs 7 and 8, the pointer 10 may be designed as a stylus shaped unit, having the light source 17 used for projecting the IOF 90 on the body 97 of the subject 98, the lock module 12, a power switch 18 for powering the pointer 10 'ON' and switching the pointer 10 'OFF', and the mechanical control module 50 which may be designed as rotating sections (not shown) of the stylus. The mechanical control module 50 may have graduations 52 or markings to assist the operator to determine how much the mechanical control module 50 is to be manipulated to achieve a desired minor adjustments in mechanical motions carried out by the movable part 91.

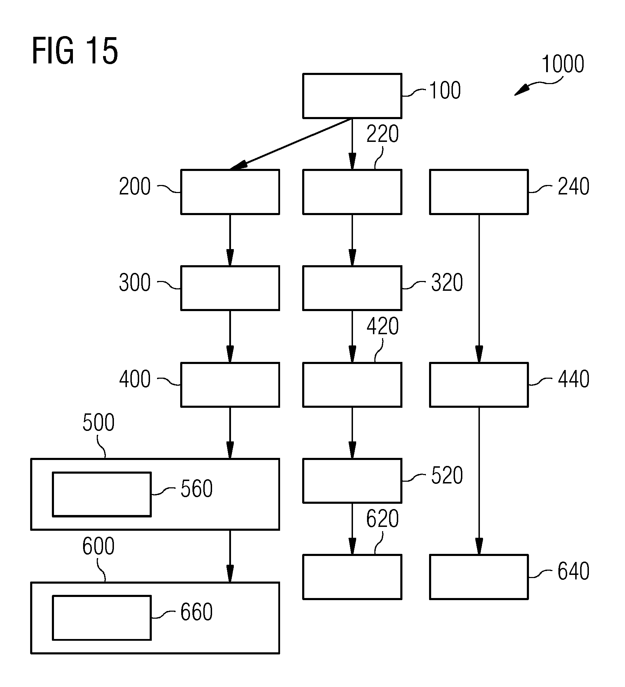

Now referring particularly to FIG 15, a flow chart depicting a method 1000 is presented. FIG 15 may be understood in combination with FIGs 1 to 14. The method 1000 is for aligning a field of view 9 of a movable part 91 of a medical device 92 with a desired region 99 on a body 97 of a subject 98. The field of view 9, the movable part 91, the medical device 92, the desired region 99, the body 97, and the subject 98 are same as explained hereinabove in reference to FIGs 1 to 14.

In the method 1000, in a step 100 an identifiable optical front 90 is projected on the body 97 of the subject 98 such that the identifiable optical front 90, hereinafter referred to the IOF 90, at least partially overlaps with the desired region 99 on the body 97 of the subject 98. The IOF 90 is same as explained hereinabove in reference to FIGs 1 to 14. In an exemplary embodiment of the method 1000, the step 100 may be performed by using the pointer 10, as explained hereinabove in reference to FIGs 1 to 14.

Subsequently, in a step 200, the IOF 90 is detected while the IOF 90 at least partially overlaps with the desired region 99 on the body 97 of the subject 98. The detection of the IOF 90 may be performed by using a position detection unit 20. The detection of the IOF 90 and the position detection unit 20, hereinafter PDU 20, is same as explained hereinabove in reference to FIGs 1 to 14.

Thereafter, in the method 1000, in a step 300, information 21 is generated. The information 21 corresponds to a spatial position 88 of the IOF 90. The information 21 is generated by the PDU 20. Subsequently, in the method 1000, in a step 400, the spatial position 88 of the IOF 90 is determined with respect to a known position 8 of the field of view 9 of the movable part 91 of the medical device 92. In an exemplary embodiment of the method 1000, the spatial position 88 of the IOF 90 is determined by using a processor 30, same as explained hereinabove with respect to FIGs 1 to 14. Furthermore, the spatial position 88, the known position 8, determination of the spatial position 88 with respect to the known position 8 are same as explained hereinabove in reference to FIGs 1 to 14.

After the step 400, in the method 1000, in a step 500, an instruction set 31 is generated. The instruction set 31 may be generated by the processor 30 and is same as the instruction set 31 explained hereinabove with respect to FIGs 1 to 14. Finally in the method 1000, in a step 600 a moving mechanism 94 of the movable part 91 is directed to carry out mechanical motions according to the instruction set 31. In an exemplary embodiment of the method 1000, the step 600 may be performed by an executing module 40. The moving mechanism 94, the executing module 40 and the directing of the moving mechanism 94 by the executing module 40 are same as explained hereinabove in reference to FIGs 1 to 14.

In an exemplary embodiment of the method 1000, the step 500 includes a step 560 in which a table-movement instruction 33 is generated to move the patient's table 96. Furthermore, in this embodiment of the method 1000, the step 600 includes a step 660 of directing a moving mechanism 66 of the patient's table 96 to carry out mechanical motions according to the table-moving instructions 33. The step 560 may be performed by the processor 30 and the step 660 may be performed by the executing module 40. The table-movement instruction 33, the moving mechanism 66, the generation of the table-movement instruction 33, and the directing of the moving mechanism 66 according to table-movement instruction 33 are same as explained hereinabove with respect to FIGs 1 to 14.

In an exemplary embodiment of the method 1000, where the IOF 90 has an extended area with the predefined shape, in a step 220, the distortion 89 in the predefined shape of the IOF 90 is detected while the IOF 90 at least partially overlaps with the desired region 99 on the body 97 of the subject 98. The step 220 may be performed by the PDU 20. Thereafter, in a step 320, the data 22 is generated. The data 22 corresponds to the distortion 89 in the predefined shape of the IOF 90. The step 320 may be performed by the processor 30. Subsequently, in the method 1000, in a step 420, the angle of illumination 85 of the IOF 90 is determined. The angle of illumination 85 is same as explained hereinabove with respect to FIGs 1 to 14. The step 420 may be performed by the processor 30. After step 420, in the method 1000 is the step 520. In the step 520, a second instruction set 32 is generated. The second instruction set 32 may be generated by the processor 30 and is same as the second instruction set 32 explained hereinabove with respect to FIGs 1 to 14. Finally, in the method 1000, in a step 620, the moving mechanism 94 of the movable part 91 is directed to carry out mechanical motions according to the second instruction set 32. The step 620 may be performed by the executing module 40.

In another embodiment of the method 1000, the method 1000 includes a step 240 of acquiring an image 25 of the body 97 with respect to a patient's table 96 of the medical device 92. The step 240 may be performed by the PDU 20. The image 25 is same as explained hereinabove with respect to FIGs 1 t 14. Subsequently, in a step 440 from the image 25, an orientation of the body 97 with respect to the patient's table 96 is determined. The step 440 may be performed by the processor 30. Finally in the method 1000, in a step 640, the moving mechanism 94 of the movable part 91 is directed to carry out mechanical motions according to the instruction set 31 when the orientation of the body 97 is a desired orientation and not otherwise. The orientations of the body 97 with respect to the patient's table 96 may be understood same as for FIGs 13 and 14.

While the present technique has been described in detail with reference to certain embodiments, it should be appreciated that the present technique is not limited to those precise embodiments. Rather, in view of the present disclosure which describes exemplary modes for practicing the invention, many modifications and variations would present themselves, to those skilled in the art without departing from the scope and spirit of this invention. The scope of the invention is, therefore, indicated by the following claims rather than by the foregoing description. All changes, modifications, and variations coming within the meaning and range of equivalency of the claims are to be considered within their scope.

## Claims

1. A system (1) for aligning a field of view (9) of a movable part (91) of a medical device (92) with a desired region (99) on a body (97) of a subject (98) by projecting an identifiable optical front (90) on the body (97) of the subject (98) such that the identifiable optical front (90) at least partially overlaps with the desired region (99) on the body (97) of the subject (98), the system (1) comprising:
- a position detecting unit (20) configured to detect the identifiable optical front (90) while the identifiable optical front (90) at least partially overlaps with the desired region (99) on the body (97) of the subject (98), wherein the position detecting unit (20) is further configured to generate information (21) corresponding to a spatial position (88) of the identifiable optical front (90);
- a processing module (30) configured to receive the information (21) from the position detecting unit (20) and to determine the spatial position (88) of the identifiable optical front (90) with respect to a known position (8) of the field of view (9) of the movable part (91) of the medical device (92), the processing module (30) is further configured to generate an instruction set (31) corresponding to one or more mechanical adjustments of the movable part (91) to change a position of the field of view (9) of the movable part (91) from the known position (8) to the spatial position (88) of the identifiable optical front (90); and
- an executing module (40) configured to receive the instruction set (31) and to direct a moving mechanism (94) of the movable part (91) to carry out mechanical motions according to the instruction set (31).

2. The system (1) according to claim 1, comprising a pointer (10) configured project the identifiable optical front (90) on the body (97) of the subject (98).

3. The system (1) according to claim 2, wherein the pointer (10) includes a lock module (12) configured to be changeable between a first state (13) and a second state (14) and wherein the pointer (10) is configured to communicate to the position detecting unit (20) a state indication and wherein the position detecting unit (20) detects the identifiable optical front (90) when the state indication from the pointer (10) communicates that the lock module (12) is in the first state (13).

4. The system (1) according to claims 2 or 3, wherein the pointer (10) comprises a spatially coherent light source (17).

5. The system (1) according to any of claims 2 to 4, wherein the identifiable optical front (90) is a point.

6. The system (1) according to any of claims 2 to 4, wherein the identifiable optical front (90) is an extended area having a predefined shape.

7. The system (1) according to claim 6, wherein:
- the position detecting unit (20) is configured to detect a distortion (89) of the predefined shape of the identifiable optical front (90) while the identifiable optical front (90) at least partially overlaps with the desired region (99) on the body (97) of the subject (98) and wherein the position detecting unit (20) is further configured to generate data (22) corresponding to the distortion (89) of the predefined shape of the identifiable optical front (90);
- the processing module (30) is configured to receive the data (22) from the position detecting unit (20) and to determine an angle of illumination (85) of the identifiable optical front (90), wherein the angle of illumination (85) corresponds to an angle, with respect to the desired region (99), from which the identifiable optical front (90) is projected on the desired region (99), and wherein the processing module (30) is further configured to generate a second instruction set (32) corresponding to one or more further mechanical adjustments of the movable part (91) to attain an aligned position (93) of the movable part (91), wherein at the aligned position (93) the movable part (91) is at an aligned angle (95) with respect to the desired region (99) and the aligned angle (95) is same as the angle of illumination (85); and
- the executing module (40) is configured to receive the second instruction set (32) and to direct the moving mechanism (94) of the movable part (91) to carry out mechanical motions according to the second instruction set (32).

8. The system (1) according to any of claims 1 to 7, wherein the position detecting unit (20), the processing module (30), and the executing module (40) are in wireless communication with each other.

9. The system (1) according to any of claims 1 to 8, wherein the position detecting unit (20) is configured to acquire an image (25) of the body (97) with respect to a patient's table (96) of the medical device (92), and wherein the processing module (30) is configured to determine from the image (25) an orientation of the body (97) with respect to the patient's table (96), and wherein the executing module (40) is configured to direct the moving mechanism (94) of the movable part (91) to carry out mechanical motions according to the instruction set (31) when the orientation of the body (97) is a desired orientation.

10. The system (1) according to any of claims 1 to 9, further including a mechanical control module (50) configured to effect minor adjustments in mechanical motions carried out by the movable part (91), wherein the mechanical control module (50) is configured to be operated by an operator.

11. The system (1) according to any of claims 1 to 10, wherein the processing module (30) is configured to generate a table-movement instruction (33) to move the patient's table (96) and the executing module (40) is configured to receive the table-movement instruction (33) and to direct a moving mechanism (66) of the patient's table (96) to carry out mechanical motions according to the table-movement instruction (33).

12. The system (1) according to any of claims 1 to 11, wherein the executing module (40) is integrated within the medical device (92).

13. A method (1000) for aligning a field of view (9) of a movable part (91) of a medical device (92) with a desired region (99) on a body (97) of a subject (98), the method (1000) comprising:
- projecting (100) an identifiable optical front (90) on the body (97) of the subject (98) such that the identifiable optical front (90) at least partially overlaps with the desired region (99) on the body (97) of the subject (98);
- detecting (200) the identifiable optical front (90) while the identifiable optical front (90) at least partially overlaps with the desired region (99) on the body (97) of the subject (98);
- generating (300) information (21) corresponding to a spatial position (88) of the identifiable optical front (90);
- determining (400) the spatial position (88) of the identifiable optical front (90) with respect to a known position (8) of the field of view (9) of the movable part (91) of the medical device (92);
- generating (500) an instruction set (31) corresponding to one or more mechanical adjustments of the movable part (91) to change a position of the field of view (9) of the movable part (91) from the known position (8) to the spatial position (88) of the identifiable optical front (90); and
- directing (600) a moving mechanism (94) of the movable part (91) to carry out mechanical motions according to the instruction set (31).

14. The method (1000) according to claim 13, wherein the identifiable optical front (90) is an extended area having a predefined shape.

15. The method (1000) according to claim 14, further comprising:
- detecting (220) a distortion (89) in the predefined shape of the identifiable optical front (90) while the identifiable optical front (90) at least partially overlaps with the desired region (99) on the body (97) of the subject (98);
- generating (320) data (22) corresponding to the distortion (89) in the predefined shape of the identifiable optical front (90);
- determining (420) an angle of illumination (85) of the identifiable optical front (90), wherein the angle of illumination (85) corresponds to an angle, with respect to the desired region (99), from which the identifiable optical front (90) is projected on the desired region (99);
- generating (520) a second instruction set (32) corresponding to one or more further mechanical adjustments of the movable part (91) to attain an aligned position (93) of the movable part (91), wherein at the aligned position (93) the movable part (91) is at an aligned angle (95) with respect to the desired region (99) and the aligned angle (95) is same as the angle of illumination (85); and
- directing (620) the moving mechanism (94) of the movable part (91) to carry out mechanical motions according to the second instruction set (32).

16. The method (1000) according to any of claims 13 to 15, further comprising:
- acquiring (240) an image (25) of the body (97) with respect to a patient's table (96) of the medical device (92);
- determining (440) from the image (25) an orientation of the body (97) with respect to the patient's table (96); and
- directing (640) the moving mechanism (94) of the movable part (91) to carry out mechanical motions according to the instruction set (31) when the orientation of the body (97) is a desired orientation.

17. The method (1000) according to claims 13 to 16, wherein generating (500) the instruction set (31) includes generating (560) a table-movement instruction (33) to move the patient's table (96); and
wherein directing (600) the moving mechanism (94) of the movable part (91) to carry out mechanical motions according to the instruction set (31) includes directing (660) a moving mechanism (66) of the patient's table (96) to carry out mechanical motions according to the table-movement instruction (33).
